# EUROPEAN PATENT APPLICATION

(11) **EP 4 435 085 A1**
(43) Date of publication of application: **25.09.2024**
(21) Application number: 23164135.8
(22) Date of filing: 24.03.2023
(51) Int. Cl.: C12M 1/36, C12M 3/00, G01N 35/00

(54) **METHOD FOR PERFORMING BIOPROCESSES ON LIQUID IMMUNE OR NAIVE CELL CULTURES**

(71) Applicant: The Automation Partnership (Cambridge) Ltd., Hertfordshire SG8 5WY (GB)
(72) Inventor: Stacey, Adrian, Royston, Hertfordshire SG8 5WY (GB); Prouté, Fanny, Royston, Hertfordshire SG8 5WY (GB)
(74) Representative: Gottschald Patentanwälte Partnerschaft mbB

(57) **Abstract**

The invention relates to a method for performing bioprocesses on liquid immune or naive cell cultures, wherein an integrated bioprocessing system (1) performs multiple unit operations (3) of a first bioprocess on a cell culture to obtain a processed cell culture, wherein the processed cell culture is destined for autologous or allogenic cell therapy, wherein a control module (7) of the integrated bioprocessing system (1) performs the unit operations (3) according to a first protocol describing a workflow (8) with order and configuration of the unit operations (3), wherein a measurement module of the integrated bioprocessing system (1) measures parameters of the performance of the unit operations (3), wherein the first protocol includes at least one branching of the workflow (8) into at least two branches, each of the branches including at least one unit operation (3), wherein, depending on the measured parameters, the control module (7) follows one of the branches. It is proposed, that the measurement module sends the measured parameters to a storage module, that the storage module stores the measured parameters in relation to the first protocol, that the stored parameters are communicated to a deciding entity for a decision about a step in a further bioprocess, that the control module (7) receives the decision from the deciding entity, and, that the control module (7) performs the further bioprocess based on the decision.

## Description

The present invention relates to a method for performing bioprocesses on liquid immune or naive cell cultures according to the general part of claim 1, to a method for performing a bioprocesses on a liquid immune or naive cell culture according to the general part of claim 13 and to an integrated bioprocessing system for performing bioprocesses on liquid immune or naive cell cultures according to the general part of claim 14.

The term "bioprocess" presently represents a biotechnological process, in particular a biotechnological process involving the use of immune cell cultures or naive cell cultures, in particular stem cell cultures. One or more processing steps might be performed on each cell culture. Hence, a bioprocess in this sense might refer to a manufacturing process that involves a sequence of processing steps performed on a cell culture which ultimately will lead to a final product.

Here, the bioprocess is in the area of cell and gene therapy, for example to manufacture autologous T cells that are modified to express a chimeric antigen receptor (CAR). These cells might be used for the treatment of various types of hematologic malignancies, including different types of leukemia (blood cancer). Other cell therapies based on naive cells, in particular stem cells and their derivates are also of interest.

For biotechnological processes involving the use of liquid immune or naive cell cultures the starting material usually is quite heterogeneous regarding its composition, for example as each donor or patient may be in a different condition (e.g., in terms of disease progression, genetic make-up or the immune system history). Therefore, certain steps of the bioprocess need to be flexibly adapted and tailored to each individual cell culture. Additionally, each cell culture may respond differently to a certain processing step applied, which requires individual adjustments within the processing step such as amounts of reagents added or the duration of incubation steps.

Hence, depending on the features of the cell culture and the bioprocess to be performed, various parameters, including for example the type, sequence or configuration of processing steps performed on each immune cell culture will need to be adjusted to the individual features of the cell culture.

In bioprocesses in the area of cell and gene therapy with allogenic or autologous production of genetically modified immune cells, compliance of the bioprocess with regulatory demands is important and the bioprocess is typically highly regulated and needs to be approved by regulatory authorities.

To ensure the safety of the product, a quality by design approach, which aims to ensure that all sources of variability affecting the bioprocess are identified, explained and managed by appropriate measures, is typically followed. This includes that all aspects related to the bioprocess are understood and controlled as much as possible. Especially, those aspects that are critical for the quality of the product need to be identified and their impact, including any deviations from their set-points, on the product need to be assessed. In the resulting design space, deviations need to be managed to keep the bioprocess compliant with the approved bioprocess. Accordingly, process data, including the type and quantity of materials used, process conditions and any deviations must be documented, and appropriate actions need to be taken in case a deviation occurs.

In a highly regulated process, as stated above, critical decisions, including how to handle a deviation, often need to be made by knowledgeable users with only a limited time-frame available for decision-making. Depending on the decision to be made, more than one user may be involved or the user may need to have a certain qualification. Additionally, the process of decision-making might be time-consuming, for example when additional documents need to be consulted. For many deviations, it is typically not foreseeable if or when they will occur. This increases the chance of human error as decisions may need to be made on short notice while being time-critical and with limited amount of information available. Additionally, the responsible users may need to be alerted first as they may not be present at all times. Accordingly, users need to be trained extensively and staff requirements are high. Additionally, a variety of measurements need to be taken, and a comprehensive and extensive documentation is mandatory to ensure the safety of the product. In this regard, user support is not just a question of working efficiently, but can also be a decisive step towards product quality and safety. Recently, more and more integrated bioprocessing systems have been proposed. Such integrated bioprocessing systems allow simultaneous performance of several bioprocesses on different cell cultures, wherein the processing steps to be performed can be adjusted according to the respective cell culture. By providing a protocol comprising a workflow for the respective bioprocesses to the integrated bioprocessing system, an automation of many unit operations becomes possible.

The known prior art (WO 2017/221155 A1) that builds the basis of the invention is related to a method according to the preamble of claim 1.

In the known method, in the context of cell and gene therapy, a control module may determine subsequent steps based on analyzing certain measured parameters of a cell culture. The control unit may for example decide to continue a further incubation of the cell culture, if a low cell number is determined.

There is a big range of possible deviations in a bioprocess and of reactions to those deviations. Even a very skilled user cannot predict every aspect of a certain reaction to a deviation. It is therefore a challenge to improve the support of a deciding entity in the case of a deviation that the protocol does not cover.

The invention is based on the problem of improving the known method such that a further optimization regarding the named challenge is reached.

The above-noted object is solved by the features of the characterizing part of claim 1.

The invention concerns a method for performing bioprocesses on liquid immune or naive cell cultures, wherein an integrated bioprocessing system performs multiple unit operations of a first bioprocess on a cell culture to obtain a first processed cell culture, wherein the first processed cell culture is destined for autologous or allogenic cell therapy, wherein a control module of the integrated bioprocessing system performs the unit operations according to a first protocol describing a workflow with order and configuration of the unit operations, wherein a measurement module of the integrated bioprocessing system measures parameters of the performance of the unit operations, wherein the first protocol includes at least one branching of the workflow into at least two branches, each of the branches including at least one unit operation, wherein, depending on the measured parameters, the control module follows one of the branches.

The main realization of the present invention is that storing measured parameters of a bioprocess to be used for decision-making in a further bioprocess is an easy way of providing a decision-making entity with context for a decision that enables making at least a partial prediction about the result of a contemplated decision based on the results of previous, similar bioprocesses.

By gathering data for a large number of bioprocesses it is possible to improve the protocols over time, to improve process knowledge and correlate clinical data with manufacturing data. If the decision-making entity is a user, there is a reduced risk of the user making an error.

In detail, it is proposed that the measurement module sends the measured parameters to a storage module, that the storage module stores the measured parameters in relation to the first protocol, that the stored parameters are communicated to a deciding entity for a decision about a step in a further bioprocess, that the control module receives the decision from the deciding entity, and, that the control module performs a further bioprocess based on the decision.

In an embodiment according to claim 2 it is proposed that the first bioprocess requires a user decision to react to a deviation. It is particularly preferred that this user action is also saved with the measured data. By saving user actions and reactions of the cell culture to the user actions in terms of the ongoing performance of the bioprocess further decisions have a better data basis and can be improved.

As detailed in claim 3 the further bioprocess may be performed according to the first protocol. Alternatively, an updated protocol may be derived (claim 4). In a particularly interesting embodiment, the branch of the protocol leading to the necessity of the user action may be eliminated in the updated protocol. It may be the case that the user action previously performed is simply repeated next time the same or a similar problem occurs.

The deciding entity may be a user and the user may be shown how the first bioprocess proceeded after a decision similar to the current decision that he needs to make (claim 5).

A suggestion module may derive a user suggestion from the stored parameters and in particular predict the performance of the further bioprocess if the user suggestion is followed (claim 6). Providing automized support to the user and at the same time still leaving the decision with the user may improve the overall performance of the integrated bioprocessing system while staying within regulatory bounds.

In an embodiment according to claim 7 the proposed method may be repeated for multiple bioprocesses thereby increasing the data basis.

The deciding entity, according to claim 8, may also be an automated decision system. Further, claim 8 relates to the possibility of including some or all modules in the integrated bioprocessing system thereby providing all information locally. Alternatively, an analysis system may be provided to process the data at another location, which is in particular advantageous if the data of multiple integrated bioprocessing systems is aggregated to improve further bioprocesses.

The protocols may, according to an embodiment of claim 9, comprise decision boundaries. Obtaining regulatory approval for an automated decision system may meet challenges. However, a protocol can be defined such that certain automated decisions may be made while staying within certain boundaries. For example, if a cell count is not high enough, the integrated bioprocessing system may automatically incubate for up to two more days. After that, a user may have to decide whether further incubation should be performed.

In an embodiment according to claim 10, the integrated bioprocessing system comprises a translation module which may translate input from the protocol into actions of the integrated bioprocessing system. It may be the case that the protocol can be used on different integrated bioprocessing systems by having different translation modules translate the protocol into the specifics of the respective integrated bioprocessing system (claims 10 and 11).

According to claim 12, an anticipation module may be provided which anticipates a deviation and informs a user prior to the deviation occurring, thereby possibly preventing the deviation or reducing its impact. The idea of anticipating a deviation is also beneficial because the qualified person can be notified to be on call or the process can be adapted in anticipation of a deviation so that the deviation is likely to occur when staff are available to handle it.

Another teaching according to claim 13, which is of equal importance, relates to a method for performing a bioprocess on a liquid immune or naive cell culture.

Here, it is essential that stored parameters of a previous bioprocess are communicated to a deciding entity for a decision about a step in the bioprocess, that the control module receives the decision from the deciding entity, and, that the control module performs the bioprocess based on the decision.

All explanations given with regard to the proposed method are fully applicable and vice versa.

Another teaching according to claim 14, which is of equal importance, relates to an integrated bioprocessing system for performing bioprocesses on liquid immune or naive cell cultures.

Here, it is essential that the measurement module sends the measured parameters to a storage module, that the storage module stores the measured parameters in relation to the first protocol,
that the stored parameters are communicated to a deciding entity for a decision about a step in a further bioprocess, that the control module receives the decision from the deciding entity, and, that the control module performs a further bioprocess based on the decision.

All explanations given with regard to the proposed first and second method are fully applicable and vice versa.

In the following, embodiments of the invention are explained with respect to the drawing. The drawing shows in
- Fig. 1,: a first embodiment of an integrated bioprocessing system,
- Fig. 2,: a second embodiment of an integrated bioprocessing system and
- Fig. 3,: a simplified workflow of a protocol.

The integrated bioprocessing system 1 shown in the drawings is preferably adapted to perform a bioprocess for the manufacturing of genetically modified T cells. Here, T cells are genetically modified to express a chimeric antigen receptor (CAR). Consequently, the term "CAR-T cells" describes T cells that have been genetically modified to express a CAR. The genetically modified CAR-T cells, which represent the product of the bioprocess, may be administered to a patient and used to start or resume cancer treatment in the patient. As the bioprocess is performed, the initial immune cell culture is gradually processed. All explanations given are mainly directed to such a bioprocess. It may be pointed out, however, that those explanations are fully applicable to other bioprocesses as well.

The term "liquid immune cell culture" is to be understood in a broad sense and refers to an immune cell culture comprising at least one type of immune cells suspended as particles in any type of liquid. As will be explained below, the liquid immune cell culture may comprise other cell types that are not immune cells. Hence, the term "liquid immune cell culture" refers to a liquid immune cell culture at any stage during the bioprocess. Consequently, the type and fraction of immune cells present in the liquid immune cell culture will change during the bioprocess applied as certain immune cells are enriched or depleted from the liquid immune cell culture and/or the immune cells are genetically modified.

The term "immune cells" generally refers to different types of white blood cells. Hence, the term "immune cells" includes a variety of cells, for example, but not limited to dendritic cells, T lymphocytes, also referred to as T cells, B lymphocytes, natural killer cells, macrophages or the like. Immune cells may also include subtypes of immune cells, for example tumor-infiltrating lymphocytes or different types of T cells. Subtypes of a certain type of immune cells may be classified based on the type of antigen present at the cell surface. Hence, the term immune cells may for example refer to T cells comprising the surface antigen CD4 ("CD4+ T cells"). Typically, a certain type of immune cells, e.g., T cells, preferably a certain subtype of immune cells, e.g., CD4+ T cells, will be selectively enriched by the bioprocess, while other immune cells, e. g. macrophages, and/or other cell types that are not immune cells, e. g., erythrocytes, and/or other subtypes of immune cells, e.g., CD8+ T cells, will be depleted from the liquid immune cell culture. The immune cells to be enriched are referred to as target immune cells, all other components to be depleted from the liquid immune cell culture are referred to "impurities". Further, and as mentioned above, the target immune cells might be genetically modified.

The term "naive cells" refers to cells that can still differentiate into different target cell types. In particular, stem cells and their derivatives prior to full differentiation into a specific cell type are naive cells. The term also comprises naive immune cells.

The term "liquid" is to be understood in a broad sense as well and refers to any liquid and/or particle-containing liquid that is processed within the integrated bioprocessing system 1. Hence, the term liquid might refer to media, waste, the liquid immune cell culture, byproducts obtained during the bioprocess, samples and/or an initial immune cell culture.

Preferably, an initial immune cell culture is obtained in a process called "leukapharesis". In leukapharesis, immune cells are obtained from the patient. Additionally or alternatively, the initial immune cell culture might also be obtained from a tissue of the patient. The initial immune cell culture might also be obtained by one or more donors that are not the patient.

Depending on the source of the initial cell culture and the bioprocess to be carried out, the initial cell culture, particularly the type, amount and distribution of impurities as well as target immune or naive cells might vary.

Presently, it is preferred that any initial immune cell culture used in the bioprocess is used for only a single patient or a small number of patients, for example up to ten patients. If that is not the case then the bioprocess presently described will yield only a cell culture for a single patient or a small number of patients and cell cultures for other patients are derived from different bioprocesses. Therefore, the proposed integrated bioprocessing system 1 is used for small scale bioprocesses.

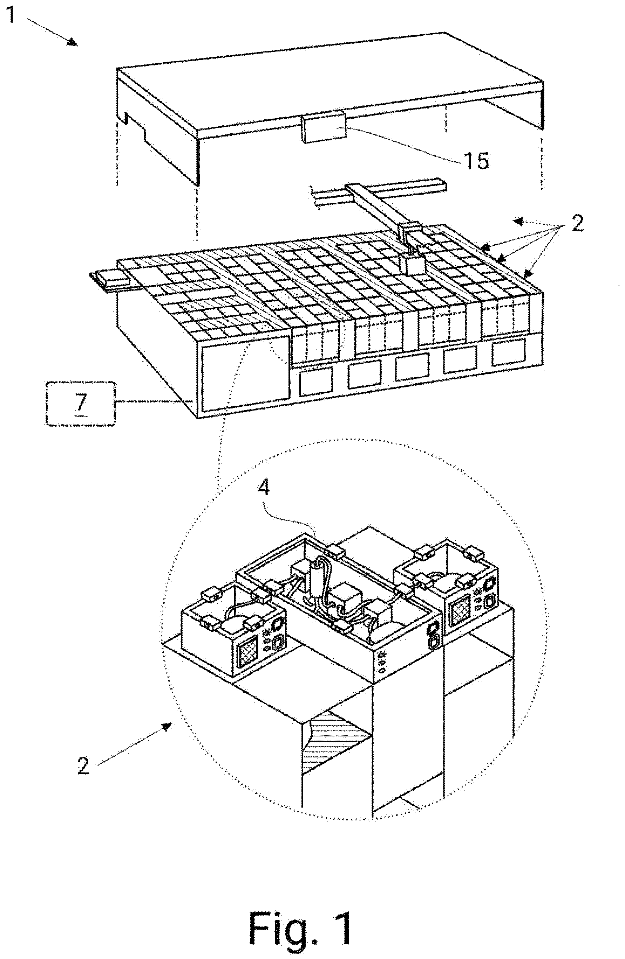

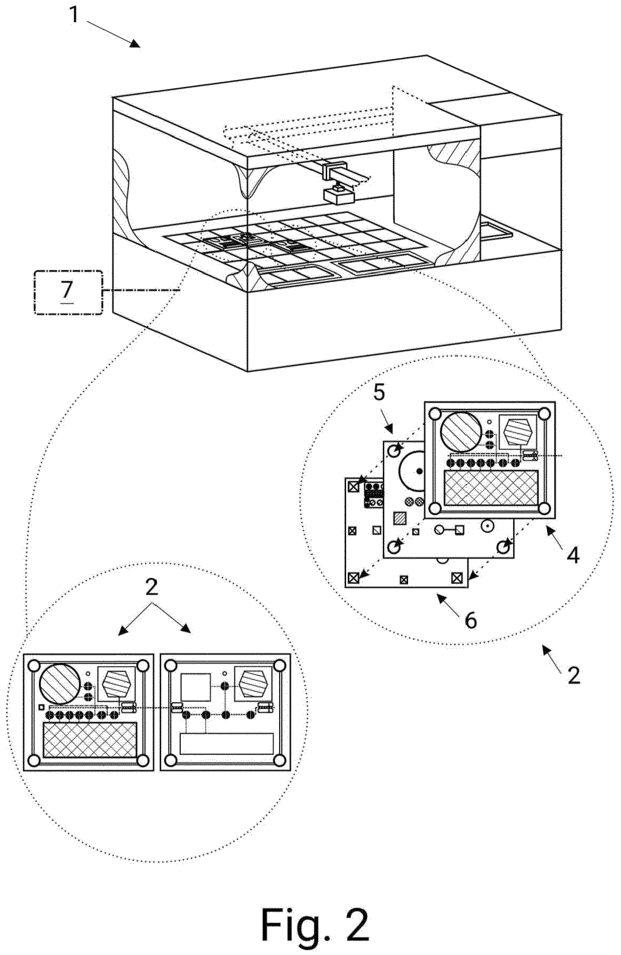

Figs. 1 and 2 show two embodiments of integrated bioprocessing systems 1. The embodiment in Fig. 1 is based on unit operation stations 2 adapted to perform one or more unit operations 3 on fluidic structures inside of cartridges 4. The embodiment of Fig. 2 is also cartridge 4 based but in this case the cartridges 4 are connected via an adapter 5 to the integrated bioprocessing system 1. The adapter 5 allows having very standardized interfaces 6 at the integrated bioprocessing system 1 and individualizing the adapters 5 for use with, in particular single-use, cartridges 4 to perform different unit operations 3. The adapters 5 may comprise individual drives, devices for the unit operations 3 and the like which are driven via standardized interfaces 6 of the integrated bioprocessing system 1 providing for example electrical energy and a data connection and possibly mechanical energy.

Proposed is a method for performing bioprocesses on liquid immune or naive cell cultures, wherein an integrated bioprocessing system 1 performs multiple unit operations 3 of a first bioprocess on a cell culture to obtain a processed cell culture, wherein the processed cell culture is destined for autologous or allogenic cell therapy. The term "first" is only included for better referencing of the bioprocess.

Unit operations 3 are basic process steps for reaching a certain physical and/or chemical change of the cell culture. Examples for common unit operations 3 are enrichment, selection, activation and genetic modification.

The integrated bioprocessing system 1 comprises a control module 7 which performs the unit operations 3 according to a first protocol describing a workflow 8 with order and configuration of the unit operations 3. A protocol may include instructions for the integrated bioprocessing system 1 like unit operations 3 to be performed, configurations of the unit operations 3 like a temperature for incubation, a speed of a centrifuge, consumables to be used and so on. The protocol comprises a workflow 8 for performing the bioprocess, including for example instructions for enrichment, selection, activation, etc., the order of those unit operations 3, possible repetitions and variations, etc. A certain type of bioprocess can be performed multiple times according to the same protocol. Different bioprocesses are usually performed according to different protocols.

In particular to control and/or monitor the bioprocess, a measurement module of the integrated bioprocessing system 1 measures parameters of the performance of the unit operations 3. The measured parameters directly or indirectly concern the cell culture. The measured parameters may comprise parameters of the cell culture like a cell count, a cell size distribution, a viability of the cell culture, a temperature of the cell culture, a density of the cell culture and the like. The parameters of the cell culture may relate to the state of the environment for the cells, in particular as monitored by sensors in varying proximity to the cells, for example a temperature of the incubator and/or temperature of the cell media. The parameters of the cell culture may include measurements taken of media containing the cells and/or of media added to the cell culture prior to the addition and/or of media removed from the cell culture. The measurements may for example be pH, dissolved oxygen, concentration measurements of media ingredients like glucose and/or metabolite concentration measurements like lactate. The parameters of the cell culture may include measurements taken of the cells of the cell culture themselves and/or measurements taken of samples from the media containing the cells and/or measurements taken of samples from the media after removing the cells.

The measured parameters may additionally or alternatively comprise parameters of the integrated bioprocessing system 1, in particular relating to a behaviour of the integrated bioprocessing system 1 and/or a state of the control system, for example a feedback from actuators of the integrated bioprocessing system 1.

The measurement module may comprise sensors anywhere in the integrated bioprocessing system 1 and processing hardware or the like.

The first protocol includes at least one branching of the workflow 8 into at least two branches, each of the branches including at least one unit operation 3, wherein, depending on the measured parameters, the control module 7 follows one of the branches.

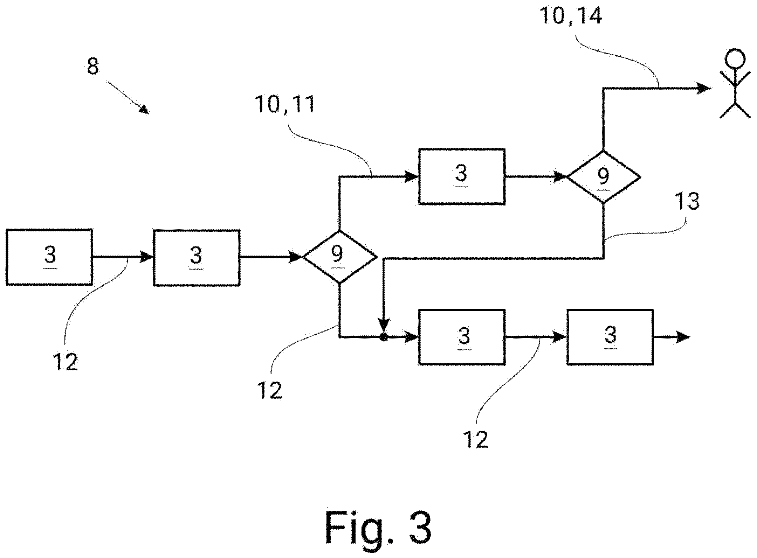

Fig. 3 shows an exemplary workflow 8 in a very simplified manner. The workflow 8 comprises several unit operations 3, several decision points 9, two of them exemplarily leading to a branching of the workflow 8 into branches. In this example, the two top branches would be deviation branches 10. At the first deviation branch 11, the control module 7 performs a unit operation 3 which may lead to reducing or removing of the deviation and subsequent returning to a standard branch 12 (returning branch 13) or to a necessary user decision (second deviation branch 14). The user decision may lead to a unit operation 3 not specified in the workflow 8, to an abortion of the protocol or to a user action that returns the workflow 8 to the standard branch 12. For example, at the second decision point 9, a cell count of the cell culture may be evaluated. If the cell count is too low, the unit operation 3 in the deviation branch 10 may be an incubation or an already running incubation may be prolonged. After the incubation, the cell count may be high enough to continue with the process or may be to low for the control module 7 to handle. Naturally, a real protocol may contain many more branches, details, different configurations of the unit operations 3, etc. Each of the branches includes at least one unit operation 3 performed when following the branch, though the unit operation 3 may be the same unit operation 3 as performed in the previous branch if a decision point 9 is located inside the timeline of a unit operation 3. As is shown, the workflow 8 may be a decision tree.

Essential is that the measurement module sends the measured parameters to a storage module and that the storage module stores the measured parameters in relation to the first protocol.

The stored parameters are further communicated to a deciding entity for a decision about a step in a further bioprocess. The further bioprocess may be a bioprocess alike to the first bioprocess, for example the same type of bioprocess performed on the same type of cell culture with the same target CAR-T cell. The further bioprocess may be performed later than the first bioprocess, even days, months or years later. The deciding entity may be a software entity or a user. The deciding entity decides and then communicates the decision to the control module 7.

The control module 7 receives the decision from the deciding entity and performs the further bioprocess based on the decision. There are two main ways of using the stored parameters that are presently in focus. The deciding entity, in particular the user, may update the first protocol, preferably prior to the start of the further bioprocess. After the update, the first protocol may be adapted to handle the same or a similar deviation without requiring a decision.

The second way relates to decisions during the performance of the further bioprocess. If a deviation occurs and a decision needs to be made, having information about similar deviations in the past and in particular about how those deviations played out after a decision, makes it easier to find a fitting decision for the further bioprocess. It may be the case that stored parameters of multiple bioprocesses performed according to the first protocol, for example at least five, are communicated to the deciding entity. The stored parameters may be processed, aggregated and generally adapted for easier decision-making.

If the modules are part of the integrated bioprocessing system 1 they may be inseparable and have overlapping program code sections. Preferably though, the modules are separate data objects.

It may be the case, that one of the branches followed by the control module 7 during the first bioprocess is a deviation branch 10 requiring a user decision, that the control module 7 receives the user decision and that the control module 7 performs a next step of the first bioprocess based on the user decision.

Preferably, the measurement module sends the user decision to the storage module and the storage module stores the user decision as one of the stored parameters. This enables comparing old user decisions with the subsequent consequences for the bioprocess and deciding based on that information. As the user decision is one of the stored parameters, it is communicated to the deciding entity. There may be further stored parameters outside the scope of the described stored parameters.

As Fig. 3 shows, it may be the case that one of the branches is a standard branch 12 and that the standard branch 12 is the quickest path for performing the bioprocess.

It may be the case that the deviation branch 10 does not lead to the end product according to the workflow 8. Rather, the deviation branch 10 may end with the decision and require help from outside the workflow 8, in particular to be reverted into a state defined in the workflow 8.

Possible deviations handled as described may include process deviations in which the cell culture itself, represented by measurement values, is outside a specification and/or system deviations in which the cell culture may be inside the specification, but the system is unable to or for other reasons does not follow the workflow 8 and needs an alternative. For example, the control module 7 may choose a unit operation 3 equivalent to the unit operation 3 planned according to the workflow 8.

As mentioned, it may be the case, that the control module 7 performs unit operations 3 of the further bioprocess according to the first protocol.

Alternatively, the user decision may be the decision about the step in the further bioprocess. For that, the decision may be included in an updated protocol and/or the protocol may be updated based on the decision. The control unit performs unit operations 3 of the further bioprocess according to the updated protocol. Preferably, the deviation branch 10 has been updated such that the same deviation does not require a user action anymore. These embodiments also show that the further bioprocess may be a specific, known bioprocess that may be currently performed or an unspecific, future bioprocess. Therefore, the deciding entity may decide during the or prior to the further bioprocess.

The updated protocol may further be updated by repeating the proposed method, thereby arriving step by step at an optimized protocol, able to deal with a multitude of deviations.

The following examples explain how the decisions and protocols may be connected.

An exemplary protocol may define that a temperature must be between 35 and 37 degrees Celsius for a successful culture. Everything outside may be a deviation. Then bioprocesses including the bioprocess of the proposed method may be run.

At some point, the temperature deviates to 37.5 degrees Celsius. The protocol may be such that a user intervention is needed at this point. The user may decide to mark this specific run (batch) of the bioprocess for a later quality check and update the protocol or the protocol may be updated automatically.

Now the protocol defines 35 - 37 degrees as normal and for 37 - 37.5 degrees that the bioprocess requires a quality check. All other temperatures may still require a user intervention.

According to one embodiment it is proposed, that the deciding entity is a user, that the stored parameters are communicated to the user by displaying them to the user. The stored parameters may be displayed by the integrated bioprocessing system 1 on a display 15 of the integrated bioprocessing system 1, preferably such that the displayed parameters show how the first bioprocess proceeded, in particular through the same branch of the same first protocol and/or after the user decision.

Expanding on the example above, it may be the case that the protocol is not updated. The next time a bioprocess runs into a temperature of for example 37.4 degrees Celsius, the user may be notified how the previous bioprocess with a deviation to 37.5 degrees turned out in the quality control. This may help the user to decide that 37.4 degrees are acceptable or not acceptable or how to proceed with the current bioprocess.

Another possibility is that a user has decided to react to a certain deviation in a certain way for a number of times and it always or never led to the expected result. The integrated bioprocessing system 1 can then inform the user about this fact and the user can decide the same or differently this time.

The example also shows that decisions can be for example adjusting a batch record to mark up an event or deviation, scheduling additional steps later in the process, e.g. an additional quality check, progress some of the material as per the original protocol and progress some of the material differently, e.g. after a selection if not enough cells are obtained in the positive fraction, that fraction may be kept and processed, but additionally the negative fraction may be processed differently to gain additional cells.

It is further possible, that a suggestion module derives a user suggestion from the stored parameters and communicates the user suggestion to the user. The suggestion may be complete or a partial suggestion, for example including only parts of a configuration of a proposed unit operation 3.

Preferably, the suggestion module predicts a performance of the further bioprocess if the user suggestion is followed and communicates the prediction to the user. The prediction may be based on similar decisions made in the past and their results and may for example be based on a statistical analysis. Also, the suggestion module may comprise a trained machine learning model trained to derive the prediction from the stored parameters. The prediction may include a quality measure indicating how secure the prediction is. For example, a prediction derived from many decisions in a very similar situation may be more secure than a prediction derived from a single remotely similar situation.

According to one embodiment it is proposed, that measured parameters and preferably user actions of multiple bioprocesses, in particular multiple bioprocesses performed according to the first protocol, are stored as the stored parameters and communicated to the deciding entity.

Preferably, the multiple bioprocesses were performed by different integrated bioprocessing systems 1, preferably different integrated bioprocessing systems 1 of the same type.

In an alternative or in addition to the user being the deciding entity in some cases, the deciding entity may be an automated decision module. It may also be the case that some less complex decisions or less impactful decisions are automated and others are presented to a user. In particular, it may be the case that the automated decision module repeats old user decisions in situations that are within a predefined similarity boundary. The repeatable decisions may be chosen for example based on their success. The automated decision system may for example use a weighting system to make a decision. Here, different scores may be assigned to at least two measured parameters and based on an overall score, a decision may be made. Exemplarily, a certain negative score may be given, if the amount of cell debris is high, whereas a certain positive score may be given, if the cell count is very low. Based on the sum of the scores, the decision may be to further incubate the culture for a certain amount of time if the sum of the scores is positive or to abort the incubation process and proceed with the next step in the workflow 8 if the sum of the scores is negative.

The storage module and/or the suggestion module and/or the automated decision module may be part of the integrated bioprocessing system 1, and/or, the storage module and/or the suggestion module and/or the automated decision module may be part of an analysis system separate from the integrated bioprocessing system 1.

According to one embodiment it is proposed, that the first protocol and/or the updated protocol comprises or comprise decision boundaries for the control module 7 and/or the automated decision module, that inside the decision boundaries the control module 7 and/or the automated decision module decides or decide how to return the bioprocess from the deviation branch 10 to a standard branch 12, and that outside the decision boundaries a user decision is required.

It may for example be the case, that the decision boundaries include a margin of 10% of the time a unit operation 3 should be performed for. The automated decision system or the control module 7 may then monitor the measured parameters and in case for example the cell count is below a certain threshold, it may consult with the stored parameters to compare whether in the past in similar situations a performance of the unit operation 3, for example an incubation, for more time has a good chance of improving the cell count.

Here and preferably, the protocol comprises unit operations 3 to be performed. The integrated bioprocessing system 1 can comprise a translation module. The translation module may automatically select components of the integrated bioprocessing system 1 and processing steps to be performed to perform the unit operation 3. Therefore, the translation module may act as a translator between a possibly generic protocol and a specific integrated bioprocessing system 1. By providing protocols which may be, at least partially, preferably completely, independent of the specific integrated bioprocessing system 1, the protocols can be improved with data from different integrated bioprocessing systems 1.

Here and preferably, the translation module selects a location for the performance of the unit operation 3 out of multiple possible locations, and/or, the translation module selects a functional element acting on the cell culture out of multiple possible functional elements. Figs. 1 and 2 show how an integrated bioprocessing system 1 may comprise multiple locations for the performance of similar or equal unit operations 3. Some of these locations are represented by the unit operation stations 2. Further, multiple functional elements, possibly different functional elements and/or locations, may be provided and chosen. The protocol may define a unit operation 3 like incubation and the control module 7 or the automated decision module may choose which type of incubation is performed and/or how and/or where.

According to one embodiment it is proposed, that the same protocol is used for differently configured integrated bioprocessing systems 1 by providing different translation modules translating the requirements of the protocol into components of the integrated bioprocessing system 1 and processing steps to be performed.

It may further be the case, that an anticipation module, in particular of the integrated bioprocessing system 1, anticipates a deviation by correlating current data of the further bioprocess with data of a previous bioprocess that led to a deviation. Anticipating deviations is particularly interesting for performing time-critical bioprocesses. Preferably, the anticipation module informs the user prior to the deviation occurring, more preferably, the anticipation module proposes a strategy for reducing or preventing the deviation.

Another teaching which is of equal importance relates to a method for performing a bioprocesses on a liquid immune or naive cell culture, wherein the integrated bioprocessing system 1 performs multiple unit operations 3 of the bioprocess on a cell culture to obtain a processed cell culture, wherein the processed cell culture is destined for autologous or allogenic cell therapy, wherein a control module 7 of the integrated bioprocessing system 1 performs the unit operations 3 according to a protocol, in particular an updated protocol, describing a workflow 8 with order and configuration of the unit operations 3, wherein a measurement module of the integrated bioprocessing system 1 measures parameters of the performance of the unit operations 3, wherein the protocol includes at least one branching of the workflow 8 into at least two branches, each of the branches including at least one unit operation 3, wherein, depending on the measured parameters, the control module 7 follows one of the branches.

It is then proposed, that stored parameters of a previous bioprocess are communicated to a deciding entity for a decision about a step in the bioprocess, that the control module 7 receives the decision from the deciding entity, and, that the control module 7 performs the bioprocess based on the decision.

The stored data here and preferably resulted from the previously described method.

Another teaching which is of equal importance relates to an integrated bioprocessing system 1 for performing bioprocesses on liquid immune or naive cell cultures, wherein the integrated bioprocessing system 1 performs multiple unit operations 3 of a first bioprocess on a cell culture to obtain a processed cell culture, wherein the processed cell culture is destined for autologous or allogenic cell therapy, wherein a control module 7 of the integrated bioprocessing system 1 performs the unit operations 3 according to a first protocol describing a workflow 8 with order and configuration of the unit operations 3, wherein a measurement module of the integrated bioprocessing system 1 measures parameters of the performance of the unit operations 3, wherein the first protocol includes at least one branching of the workflow 8 into at least two branches, each of the branches including at least one unit operation 3, wherein, depending on the measured parameters, the control module 7 follows one of the branches.

It is then proposed, that the measurement module sends the measured parameters to a storage module, that the storage module stores the measured parameters in relation to the first protocol, that the stored parameters are communicated to a deciding entity for a decision about a step in a further bioprocess, that the control module 7 receives the decision from the deciding entity, and, that the control module 7 performs the further bioprocess based on the decision.

## Claims

1. Method for performing bioprocesses on liquid immune or naive cell cultures, wherein an integrated bioprocessing system (1) performs multiple unit operations (3) of a first bioprocess on a cell culture to obtain a processed cell culture, wherein the processed cell culture is destined for autologous or allogenic cell therapy, wherein a control module (7) of the integrated bioprocessing system (1) performs the unit operations (3) according to a first protocol describing a workflow (8) with order and configuration of the unit operations (3),
wherein a measurement module of the integrated bioprocessing system (1) measures parameters of the performance of the unit operations (3), wherein the first protocol includes at least one branching of the workflow (8) into at least two branches, each of the branches including at least one unit operation (3), wherein, depending on the measured parameters, the control module (7) follows one of the branches,
**characterized in**
**that** the measurement module sends the measured parameters to a storage module, that the storage module stores the measured parameters in relation to the first protocol,
**that** the stored parameters are communicated to a deciding entity for a decision about a step in a further bioprocess, that the control module (7) receives the decision from the deciding entity, and, that the control module (7) performs the further bioprocess based on the decision.

2. Method according to claim 1, **characterized in that** one of the branches followed by the control module (7) during the first bioprocess is a deviation branch (10) requiring a user decision, that the control module (7) receives the user decision, that the control module (7) performs a next step of the first bioprocess based on the user decision, preferably, that the measurement module sends the user decision to the storage module, that the storage module stores the user decision as one of the stored parameters.

3. Method according to claim 1 or 2, **characterized in that** the control module (7) performs unit operations (3) of the further bioprocess according to the first protocol.

4. Method according to claim 2, **characterized in that** the user decision is the decision about the step in the further bioprocess, preferably, that the user decision is included in an updated protocol and the control unit performs unit operations (3) of the further bioprocess according to the updated protocol, more preferably, that the deviation branch (10) has been updated such that the same deviation does not require a user action anymore.

5. Method according to one of the preceding claims, **characterized in that** the deciding entity is a user, that the stored parameters are communicated to the user by displaying them to the user, in particular by the integrated bioprocessing system (1) on a display (15) of the integrated bioprocessing system (1), preferably such that the displayed parameters show how the first bioprocess proceeded, in particular through the same branch of the same first protocol and/or after the user decision.

6. Method according to one of the preceding claims, **characterized in that** a suggestion module derives a user suggestion from the stored parameters and communicates the user suggestion to the user, preferably, that the suggestion module predicts a performance of the further bioprocess if the user suggestion is followed and communicates the prediction to the user, more preferably, that the suggestion module comprises a trained machine learning model trained to derive the prediction from the stored parameters.

7. Method according to one of the preceding claims, **characterized in that** measured parameters and preferably user actions of multiple bioprocesses, in particular multiple bioprocesses performed according to the first protocol, are stored as the stored parameters and communicated to the deciding entity, preferably, that the multiple bioprocesses were performed by different integrated bioprocessing systems (1).

8. Method according to one of the preceding claims, **characterized in that** the deciding entity is an automated decision module, and/or, that the storage module and/or the suggestion module and/or the automated decision module is or are part of the integrated bioprocessing system (1), and/or, that the storage module and/or the suggestion module and/or the automated decision module is or are part of an analysis system separate from the integrated bioprocessing system (1).

9. Method according to one of the preceding claims, **characterized in that** the first protocol and/or the updated protocol comprises or comprise decision boundaries for the control module (7) and/or the automated decision module, that inside the decision boundaries the control module (7) and/or the automated decision module decides or decide how to return the bioprocess from the deviation branch (10) to a standard branch (12), and that outside the decision boundaries a user decision is required.

10. Method according to one of the preceding claims, **characterized in that** the protocol comprises unit operations (3) to be performed, that the integrated bioprocessing system (1) comprises a translation module, that the translation module automatically selects components of the integrated bioprocessing system (1) and processing steps to be performed to perform the unit operation (3), preferably, that the translation module selects a location for the performance of the unit operation (3) out of multiple possible locations, and/or, that the translation module selects a functional element acting on the cell culture out of multiple possible functional elements.

11. Method according to one of the preceding claims, **characterized in that** the same protocol is used for differently configured integrated bioprocessing systems (1) by providing different translation modules translating the requirements of the protocol into components of the integrated bioprocessing system (1) and processing steps to be performed.

12. Method according to one of the preceding claims, **characterized in that** an anticipation module, in particular of the integrated bioprocessing system (1), anticipates a deviation by correlating current data of the further bioprocess with data of a previous bioprocess that led to a deviation, preferably, that the anticipation module informs the user prior to the deviation occurring, more preferably, that the anticipation module proposes a strategy for reducing or preventing the deviation.

13. Method for performing a bioprocesses on a liquid immune or naive cell culture, wherein the integrated bioprocessing system (1) performs multiple unit operations (3) of the bioprocess on a cell culture to obtain a processed cell culture, wherein the processed cell culture is destined for autologous or allogenic cell therapy,
wherein a control module (7) of the integrated bioprocessing system (1) performs the unit operations (3) according to a protocol, in particular an updated protocol, describing a workflow (8) with order and configuration of the unit operations (3), wherein a measurement module of the integrated bioprocessing system (1) measures parameters of the performance of the unit operations (3), wherein the protocol includes at least one branching of the workflow (8) into at least two branches, each of the branches including at least one unit operation (3), wherein, depending on the measured parameters, the control module (7) follows one of the branches,
**characterized in**
**that** stored parameters of a previous bioprocess are communicated to a deciding entity for a decision about a step in the bioprocess, that the control module (7) receives the decision from the deciding entity, and, that the control module (7) performs the bioprocess based on the decision.

14. Integrated bioprocessing system for performing bioprocesses on liquid immune or naive cell cultures, wherein the integrated bioprocessing system (1) performs multiple unit operations (3) of a first bioprocess on a cell culture to obtain a processed cell culture, wherein the processed cell culture is destined for autologous or allogenic cell therapy,
wherein a control module (7) of the integrated bioprocessing system (1) performs the unit operations (3) according to a first protocol describing a workflow (8) with order and configuration of the unit operations (3),
wherein a measurement module of the integrated bioprocessing system (1) measures parameters of the performance of the unit operations (3), wherein the first protocol includes at least one branching of the workflow (8) into at least two branches, each of the branches including at least one unit operation (3), wherein, depending on the measured parameters, the control module (7) follows one of the branches,
**characterized in**
**that** the measurement module sends the measured parameters to a storage module, that the storage module stores the measured parameters in relation to the first protocol,
**that** the stored parameters are communicated to a deciding entity for a decision about a step in a further bioprocess, that the control module (7) receives the decision from the deciding entity, and, that the control module (7) performs the further bioprocess based on the decision.
